# FASCICULE DE BREVET EUROPEEN

(11) **EP 2 265 723 B1**
(45) Date de publication et mention de la délivrance du brevet: **18.07.2012**
(21) Numéro de dépôt: 08865165.8
(22) Date de dépôt: 15.12.2008
(51) Int. Cl.: C12P 7/46, C12R 1/19, C07C 51/43, C07C 51/41, C07C 51/02

(54) **Procédés de production d'acide succinique**
Verfahren zur Herstellung von Bernsteinsäure
Methods for producing succinic acid

(30) Priorité: 13.12.2007 FR 0759827; 18.02.2008 FR 0851028
(43) Date de publication de la demande: 29.12.2010
(60) Demande divisionnaire: 11172720.2
(73) Titulaire: Roquette Freres, 62136 Lestrem (FR)
(72) Inventeur: DEHAY, Frédéric, F-62840 Laventie (FR); SEGUEILHA, Laurent, F-59350 Saint Andre Lez Lille (FR); CALANDE, Olivier, F-59280 Armentieres (FR); VARLAMOFF, Caroline, F-59112 Annoeulin (FR)
(74) Mandataire: Cabinet Plasseraud
(86) Numéro de dépôt international: PCT/FR2008/052300
(87) Numéro de publication internationale: WO 2009/081012

(56) Documents cités:
- EP-A- 0 389 103
- EP-A1- 0 405 707
- WO-A1-99/09196
- WO-A1-2007/046389
- JP-A- 2005 333 886
- US-A1- 2005 042 736
- US-A1- 2006 046 288
- SANCHEZ A M ET AL: "Novel pathway engineering design of the anaerobic central metabolic pathway in Escherichia coli to increase succinate yield and productivity" METABOLIC ENGINEERING, ACADEMIC PRESS, US, vol. 7, no. 3, 1 mai 2005 (2005-05-01), pages 229-239, XP004879518 ISSN: 1096-7176 cité dans la demande
- HONG W H ET AL: "Method for purifying succinic acid from fermented solution with high efficiency and low cost" WPI/THOMSON,, vol. 2007, no. 25, 21 juillet 2006 (2006-07-21), XP002494388 & KR 2006 0083729 A (KOREA INST SCIENCE TECHNOLOGY [KR]) 21 juillet 2006 (2006-07-21)
- DATABASE BIOSIS [Online] BIOSCIENCES INFORMATION SERVICE, PHILADELPHIA, PA, US; septembre 2006 (2006-09), ISAR JASMINE ET AL: "Effect of process parameters on succinic acid production in Escherichia coli W3110 and enzymes involved in the reductive tricarboxylic acid cycle" XP002577692 Database accession no. PREV200700025149 & CANADIAN JOURNAL OF MICROBIOLOGY, vol. 52, no. 9, septembre 2006 (2006-09), pages 893-902, ISSN: 0008-4166

## Description

La présente invention concerne des procédés de production d'acide succinique et/ou d'ions succinate par fermentation en conditions anaérobies.

L'acide succinique (ou acide butanedioïque) est un acide organique avec deux groupes carboxyles, de formule semi-développée COOH-CH₂-CH₃-COOH, qui intervient dans le métabolisme cellulaire, comme intermédiaire métabolique du cycle de Krebs dans la mitochondrie.

Il trouve de nombreuses applications dans les domaines cosmétiques, agroalimentaires, pharmaceutiques, textiles et dans les plastiques. Ainsi il est par exemple utilisé comme intermédiaire de synthèse des plastiques pour la fabrication de 1,4 butanediol, de tétrahydrofuranne et de gammabutyrolactone.

De nouveaux produits dérivés de l'acide succinique sont en constant développement, incluant le développement de polyesters.

Généralement, les esters de l'acide succinique ont le potentiel d'être de nouveaux solvants "verts" qui peuvent substituer les servants plus nuisibles pour l'homme et l'environnement.

La production d'acides carboxyliques, tels que l'acide malique, l'acide succinique ou l'acide fumarique, a partir de matières premières renouvelables (en l'occurrence par le biais des processus de fermentation) est connue de l'Homme du métier.

Le succinate est un intermédiaire métabolique lors de la fermentation anaérobie par des bactéries produisant du propionate, mais ces procédés de fermentation résultent en la production de très faibles rendements et titres en acide succinique.

Ces dernières années, beaucoup de microorganismes producteurs d'acide succinique ont été isolés, comme par exemple les bactéries anaérobies du rumen, *Bacteroides ruminicola* et *Bacteroides amylophilus.* Toutefois, les organismes du Rumen sont très instables lors des procédés de fermentations, et ne sont donc pas utilisables de manière industrielle pour la production d'acide succinique.

On sait depuis longtemps qu'un mélange de plusieurs acides, dont l'acide succinique est produit à partir de la fermentation de *E. coli* en présence de glucose et de CO₂ comme substrats carbonés, comme décrit par JL Stokes en 1949 « Fermentation of glucose by suspensions of Escherichia coli » J. Bacteriol., 57; 147-158. Cependant, pour chaque mole de glucose fermentée, seule 0.3 à 0.4 Moles d'acide succinique sont produites. Des études ont donc été menées sur des bactéries, en particulier *Escherichia coli*. modifiées génétiquement de manière à inactiver les voies métaboliques consommant le NADH nécessaire à la fabrication de l'acide succinique et de manière à activer les voies métaboliques de production du succinate (sel de l'acide succinique).

En effet, le chemin métabolique fermentaire qui permet la conversion de l'oxaloacétate en malate, puis fumarate et enfin succinate nécessite deux moles de NADH par mole de succinate produit. Le verrou métabolique majeur pour la production de succinate et donc la biodisponibilité cellulaire en NADH.

Pour solutionner cette difficulté, le document US 7.223.567 décrit l'utilisation d'une souche *Escherichia coli* recombinante surproductrice de succinate pour la même quantité de NADH disponible.

Cette souche *Escherichia coli* SBS550 MG pHL 413 présente une inactivation des produits des gènes *adhE*, *ldhA* (impliqués dans les voies consommatrices de NADH), et une inactivation des produits des gènes *ack-pta* et du gène *iclR* (activant la voie glyoxylate), et contient un vecteur plasmidique surexprimant un gène PYC exogène. L'article de Sanchez et al. (intitulé « Novel pathway engineering design of the anaerobic central metabolic pathway in Escherichia coli to increase succinate yield and productivity » in Metabolic Engineering 7 (2005) 229-239), le brevet américain US 7 223 567 et la demande de brevet américain US 2005/0042736 ont développé de nouvelles conditions de culture et de production associées à cette souche pour améliorer ses rendements de production en acide succinique.

L'homme du métier recherché constamment de nouveaux procédés améliorés de production d'acide succinique. En particulier, l'homme du métier cherche à optimiser le rendement et la productivité obtenus. Par ailleurs, les méthodes de fermentation classiques conduisent à d'importants rejets en dioxyde de carbone dans l'atmosphère, ce qui est bien évidemment peu désirable.

Selon un aspect, la présente invention concerne un procédé de production d'acide succinique et/ou d'ions succinate par fermentation anaérobie d'une souche d'*Escherichia coli*, comprenant :
(A) une étape de fermentation d'une source de carbone dans un fermenteur, avec apport de CO₂, réalisée avec évents du fermenteur fermés de manière à ce que l'apport de CO₂, soit asservi à la consommation de CO₂ par la souche ; suivie, avant épuisement total de la source de carbone, d'
(B) une étape de fermentation de la source de carbone restante, sans apport de CO₂, de manière à consommer le CO₂ résiduel.

L'homme du métier est familier avec les techniques de fermentation (telles que notamment décrite dans Fermentation & Biochemical Engineering Handbook : princip/es, process design & equipment, 2nd ed 1996 de VOGEL Henry C. et TODARO Celeste L.).

La fermentation est une réaction biochimique qui consiste généralement à libérer de l'énergie, ou à produire certains métabolites d'intérêt à partir d'un substrat organique sous l'action d'enzymes microbiennes.

La fermentation est généralement conduite dans des dispositifs (fermenteurs) adaptés au processus de fermentation, c'est-à-dire adaptés à la culture de microorganismes dans les conditions voulues (dispositifs permettant le cas échéant de contrôler les équilibres gazeux du milieu de culture, notamment par des conduits d'entrée et/ou de sortie en gaz, évents, etc ; dispositifs permettant l'introduction de milieu de culture et autres substances ; dispositifs permettant de contrôler, réguler, modifier d'autres types de paramètres, tels que l'agitation, la température, le pH, etc).

L'homme du métier est également familier avec la fermentation en conditions anaérobies. Selon la présente invention, ceci désigne des conditions de culture en l'absence d'oxygène. De manière préférée, des conditions de culture anaérobies sont des conditions de culture en présence de dioxyde de carbone. Selon un mode de réalisation, les conditions de fermentation anaérobies en présence de CO₂ et/ou avec apport de CO₂ sont des conditions de fermentation en saturation de CO₂.

Par « avant épuisement total de la source de carbone, » lors de l'étape (A), on entend un instant de l'étape (A) où le milieu de fermentation contient une quantité résiduelle en source de carbone pouvant être intégralement transformée par la souche en acide succinique et/ou succinate grâce au CO₂ disponible en solution a cet instant (sous forme de CO₂, dissous ou de HCO₃⁻).

Lors de l'étape (A), le(s) évent(s) du fermenteur étant fermé(s) et l'alimentation en CO₂ du fermenteur étant maintenue, l'apport de CO₂ est réalisé de manière discontinue, automatiquement ajusté en fonction de la consommation du CO₂ par la souche pour la production d'acide succinique et/ou succinate.

Par « automatiquement ajusté », on entend, compte tenu de l'équilibre thermodynamique entre la phase liquide (milieu de fermentation) et la phase gazeuse (« atmosphère ») présentes dans le fermenteur (évent(s) fermé(s)), apport d'une quantité donnée de CO₂ ne peut intervenir que suite à la consommation d'une quantité équivalente par la souche par fermentation (et donc la production concomitante d'acide succinique et/ou succinate).

Lors de l'étape (B), il n'y a pas d'apport de CO₂, ce qui signifie que l'apport en CO₂ réalisée lors de l'étape (A) est interrompu. Ceci peut notamment être réalisé par coupure de l'alimentation en CO₂.

Avantageusement selon l'invention, la fermentation lors de l'étape (B) consomme le CO₂ (résiduel dissous) et les ions HCO₃⁻ présents dans le milieu de fermentation.

Selon un mode préféré de réalisation, avant le début de l'étape (A), l'apport de CO₂ est réalisé par injection, avec évents du fermenteur ouverts, de manière à atteindre la saturation du milieu de fermentation en CO₂

A titre d'exemple, le CO₂ peut être introduit par injection à un débit de 0,15-0,40 vvm (volume de CO₂ par volume de culture par minute), de préférence 0,3 vvm.

Par « milieu de fermentation saturé en CO₂ », on entend le fait que le milieu de culture contient la quantité maximale en CO₂ qui peut y être dissous dans les conditions correspondantes (température, pH, etc). Par exemple ceci peut correspondre à une concentration de 1-2 g/L, par exemple de l'ordre de 1,5 g/L a 37°C, pH 7.

Selon un mode de réalisation, l'étape (A) et/ou l'étape se déroule à un pH dans un intervalle de 6,0-7,0, de préférence 6,4-6,8, de préférence 6,5-6,6.

Selon un mode de réalisation, la source de carbone est le glucose.

Selon un mode de réalisation, au début de l'étape (A), le milieu de fermentation comprend 15-40 g/L, de préférence 15-25 g/L, de préférence 15-20 g/L de glucose. Selon une mode de réalisation, au début de l'étape (B), le milieu de fermentation comprend 2-6 g/L, de préférence environ 4 g/L de glucose.

Selon un mode de réalisation préféré, la souche d'*Escherichia Coli* est une souche qui possède le génotype Δ*adhE* Δ*ldhA* Δ*icl*R Δ*ackpta* PYC. Ce génotype permet avantageusement de favoriser la production d'acide succinique par fermentation en présence de CO₂, Le symbole Δ indique que le gène en question a été inactivé, par exemple par mutation, délétion, interruption, insertion, ou « down »-régulation, par exemple par introduction d'un codon stop, insertion ou délétion résultant en un changement du cadre de lecture, mutation ponctuelle, etc.

Le génotype Δ*adhE* Δ*ldhA* Δ*iclR* Δ*ackpta* PYC correspond donc à :
- Δ*adhE* : inactivation de l'alcool deshydrogénase ;
- Δ*ldhA* : inactivation de la lactate déshydrogénase ;
- Δ*iclR* : inactivation de l'isocitrate lyase (également connue sous le nom de aceA)
- Δ*ackpta* : inactivation de l'acétate kinase-phosphotransacétylase ;
- PYC ; expression d'un gène de pyruvate carboxylase. Ceci indique que la souche exprime le gène PYC, par exemple grâce à une transformation par un plasmide portant un exemplaire fonctionnel de ce gène, ou par intégration génomique d'un exemplaire fonctionnel de PYC. Le gène PYC est avantageusement le gène pyc de *Lactococcus lactis.*

Selon un mode de réalisation très préféré, la souche d'*Escherichia Coli* est la souche SBS550MG - pHL413. Cette souche est décrite dans Sanchez et al., Metabolic Engineering, 7 (2005) 229-239, et dans les documents US 7,223,567 et US 2005/0042736.

Selon un autre aspect la présente invention concerne un procédé d'obtention d'acide succinique comprenant :
- un procédé de production d'acide succinique et/ou d'ions succinate tel que décrit ci-dessus ;
- le cas échéant, une étape d'acidification des ions succinate en acide succinique (par exemple par ajout d'acide tort dans le moût),
- une étape de purification de l'acide succinique ; et optionnellement une étape de cristallisation de l'acide succinique.

Selon un mode de réalisation, dans tous les procédés décrits ci-dessus, l'étape de purification comprend une purification éthanolique qui se déroule comme suit :
- filtration (élimination d'un précipité protéique), par exemple sur Büchner et/ou sur terre de filtration du mout acidifié,
- optionnellement, concentration du filtrat par évaporation sous vide (de préférence, suivant un facteur de concentration entre environ 2 et 8),
- ajout d'éthanol, par exemple de l'éthanol 95%, dans un rapport 1/1 à 5/1, pour provoquer la précipitation des sels (l'acide succinique reste soluble),
- séparation du précipité salin par filtration, par exemple sur une membrane,
- récupération de l'éthanol par évaporation sous vide,
- traitement sur charbon actif, puis filtration sur plaque et terre de filtration,

L'invention est illustrée par les exemples de réalisation ci-dessous, qui sont non-limitatifs.

### Exemples

### Exemple 1 : Production d'acide succinique par fermentation anaérobie selon deux modes différents d'apport du CO₂ avec évent du fermenteur ouvert ou fermé

Le procédé de production d'acide succinique comprend :
- une phase de préculture en Erlenmeyer,
- une phase de culture en conditions aérobies dans un milieu de culture comprenant du corn steep comme source d'azote et du glucose comme source de carbone, cette phase permettant la production de biomasse, et
- une phase anaérobie permettant la production proprement dite en acide succinique. Les phases en conditions aérobie et anaérobie sont réalisées dans un même fermenteur. La souche utilisée est la souche SBS550MG-pHL413.

### Phase aérobie :

La souche SBS550MG-pHL413 est précultivée en Erlenmeyer pendant 17h à 37°C, sous agitation à 125 rpm. 400 ml de milieu sont inoculés par la souche dans un Erlenmeyer de 2 litres à 2 chicanes.

La composition de ce milieu de préculture est la suivante:

| | |
|---|---|
| tryptone | 10g/L |
| extrait de levure | 5g/L |
| NaCl | 10 g/L |
| Antibiotiques (ampicilline, carbenicilline, oxacilline) | 67 mg/L |

La souche ainsi précultivée est placée dans un fermenteur de 15L dans un milieu de culture dont la composition est la suivante :

| **Glucose :** 2 g/L départ + 2 g/L lorsque les premiers 2g/L sont consommés | |
|---|---|
| **Corn steep :** 60 g/L | |
| **Sels et antibiotiques :** | g/L |
| (NH₄)₂SO₄ | 0,25 |
| K₂HPO₄ | 0,7 |
| KH₂PO₄ | 1,2 |
| KCl | 2 |
| CaCl₂ | 0,2 |
| MgSO₄ | 0,25 |
| Ampicilline | 0,067 |
| Biotine Thiamine | 0.001 |

L'inoculum obtenu par préculture en Erlenmeyer représente 3% du volume total du milieu cultivé dans le fermenteur.

Les conditions de culture lors de la phase aérobie sont une température de 37°C, une agitation de 500 rpm, une aération de 1 vvm et pas de régulation de pH (le pH est simplement ajusté à 7,5 avant stérilisation du milieu).

### Phase anaérobie:

### o Protocole avec apport continu de CO₂

- On ajoute dans le milieu: glucose: 20 g/L départ + 15 g/L à 24h + 4 g/L à 50h.
- La fermentation est réalisée à pH 6,4 avec une injection continue de CO₂ à un débit de 03 vvm (L/L/min), à 37°C, évent du fermenteur ouvert, sous agitation 250 rpm.
- Elle se termine en 63,5 h avec un titre final en acide succinique de 30 g/L dans le milieu de culture.
- La quantité globale de CO₂ consommée s'établit à (0,3 vvm x 60 min x 63,3 h / 22,4 mol/L x 44 g/mol) 2245 g/L, soit 73 g/g d'acide succinique formé.
- Par ailleurs, une concentration de 2 g/L d'HCO₃ (correspondant à 1,5 g/L de CO₂) est présente en fin de fermentation dans le milieu de culture.

### o Protocole selon l'invention

Le protocole de fermentation est identique à celui ci-dessus, excepté que
- au début de la phase anaérobie, du CO₂ est introduit dans le fermenteur à un débit de 0.3 vvm pendant 1 minute de façon à chasser l'air résiduel issu de la phrase aérobie,
- la pression du réseau de CO₂ est réduite à 0,4 bar,
- l'évent du fermenteur est fermé hermétiquement de façon à empêcher la sortie du CO₂,
- ainsi, l'injection de CO₂ est précisément ajustée à sa consommation tout au long de la fermentation,
- l'injection de CO₂ est coupée lorsque la concentration résiduelle en glucose atteint 4 g/L de façon à consommer l'HCO₃⁻ résiduel dissous dans le milieu.

### Résultats selon l'invention

- concentration finale en acide succinique produit dans le milieu de culture en fin de fermentation : 30 g/L, ce qui est identique à la concentration obtenue avec apport continu en CO₂.
- concentration de HCO₃⁻ résiduel dans le milieu de culture en fin de fermentation : 0,3 g/L (ce qui représente une réduction de 85% par rapport à la concentration obtenue avec apport continu en CO₂).
- consommation de CO₂ : 0,59 g/l au départ + 6 g/l fixé sur le succinique, soit 0,2 g / g d'acide (ce qui représente une réduction de 99,7% par rapport à la concentration obtenue avec apport continu en CO₂)

Ainsi, avantageusement selon l'invention, tout en maintenant le rendement de production d'acide succinique, on évite d'importants rejets en dioxyde de carbone :
- le fait de travailler en réacteur clos limite naturellement les rejets, et
- par ailleurs, on observe une diminution de la concentration en HCO₃⁻ résiduel dans le milieu de culture en fin de fermentation. Or, l'acidification de l'HCO₃⁻ résiduel dans le milieu de culture en fin de fermentation conduit à un dégagement de CO₂.

### Exemple 2 : Obtention d'acide succinique par fermentation anaérobie, purification éthanolique et cristallisation

Le procédé de production d'acide succinique de l'Exemple 1 (avec NaOH comme agent de régulation du pH) est suivi d'une étape de purification éthanolique comme décrit ci-dessous;
- centrifugation du milieu de fermentation (moût) (5000g, 15', 20°C) (élimination de la biomasse),
- ajout d'acide sulfurique 95% au surnageant jusqu'à obtention d'un pH 1,5,
- filtration du moût sur Büchner avec plaque SEITZ EK et terre de filtration FW20 (élimination d'un précipité protéique),
- concentration du filtrat par évaporation sous vide (facteur de concentration oscille entre environ 2 et 8),
- ajout d'éthanol 95%, 2 volumes d'éthanol pour 1 volume du filtrat concentré pour provoquer la précipitation des sels (l'acide succinique reste soluble),
- séparation du précipité salin par filtration sur une membrane millipore 3 microns,
- récupération de l'éthanol par évaporation sous vide.
- traitement charbon actif (2%/sec de PUREFLOW C - 80°C - 1 heure) puis filtration sur plaque SElTZ EK et terre de filtration FW20,
- évapocristallisation (Tp bain marie 75°c - Pression résiduelle 60 mbars MS masse cuite environ 50%),
- refroidissement de la masse cuite sous agitation à 20°C,
- séparation des cristaux par filtration sur une membrane millipore 3 microns,
- clairçage des cristaux avec de l'eau déminéralisée,
- séchage des cristaux une nuit à 60°c sous vide.

## Revendications

1. Procédé de production d'acide succinique et/ou d'ions succinate par fermentation en conditions anaérobies d'une souche d'*Escherichia coli,* comprenant :
(A) une étape de fermentation d'une source de carbone dans un fermenteur, avec apport de CO₂, réalisée avec évents du fermenteur fermés de manière à ce que l'apport de CO₂ soit asservi à la consommation de CO₂ par la souche ; suivie, avant épuisement total de la source de carbone, d'
(B) une étape de fermentation de la source de carbone restante, sans apport de CO₂ , de manière à consommer le CO₂ résiduel.

2. Procédé selon la revendication 1, dans lequel, au début de l'étape (A), le milieu de fermentation est saturé en CO₂.

3. Procédé selon l'une quelconque des revendications 1-2 dans lequel l'étape (A) et/ou l'étape (B) se déroule à un pH dans un intervalle de 6,0-7,0.

4. Procédé selon l'une quelconque des revendications 1-2 dans lequel l'étape (A) et/ou l'étape (B) se déroule à un pH de 6,4-6,8..

5. Procédé selon l'une quelconque des revendications 1-4, dans lequel la source de carbone est le glucose, et dans lequel :
- au début de l'étape (A), le milieu de fermentation comprend 15-40 g/L de glucose, et
- au début de l'étape (B), le milieu de fermentation comprend 2-6 g/L de glucose.

6. Procédé selon l'une quelconque des revendications 1-5, dans lequel la souche d'*Escherichia Coli* possède le génotype Δ*adhE* Δ*ldh4* Δ*iclR* Δ*ackpta* PYC.

7. Procédé selon la revendication 6, **caractérisée en ce que** la souche d'*Escherichia coli* est la souche SBS550MG - pHL413.

8. Procédé d'obtention d'acide succinique, comprenant :
- un procédé de production d'acide succinique et/ou d'ions succinate selon l'une quelconque des revendications 1-7 ;
- le cas échéant, une acidification des ions succinate en acide succinique ;
- une étape de purification de l'acide succinique; et
- optionnellement, une étape de cristallisation de l'acide succinique.

9. Procédé d'obtention d'acide succinique selon la revendication 8, **caractérisée en ce que** l'étape de purification de l'acide succinique est une purification éthanolique.

## Claims

1. A method for producing succinic acid and/or succinate ions by fermentation under anaerobic conditions of an *Escherichia coli* strain, comprising:
(A) a step of fermentation of a carbon source in a fermenter, with CO₂ being supplied, carried out with fermenter vents closed such that the supply of CO₂ is controlled by the consumption of CO₂ by the strain; followed, before complete depletion of the carbon source, by
(B) a step of fermentation of the remaining carbon source, without CO₂ being supplied, so as to consume the residual CO₂.

2. The method according to claim 1, in which, at the start of step (A), the fermentation medium is saturated with CO₂.

3. The method according to any one of claims 1 and 2, in which step (A) and/or step (B) is carried out at a pH within a range of 6.0-7.0.

4. The method according to any one of claims 1 and 2, in which step (A) and/or step (B) is carried out at a pH within a range of 6.4-6.8

5. The method according to any one of claims 1-4, in which the carbon source is glucose, and in which:
- at the start of step (A), the fermentation medium comprises 15-40 g/l of glucose, and
- at the start of step (B), the fermentation medium comprises 2-6 g/l of glucose.

6. The method as claimed in any one of claims 1-5, in which the *Escherichia coli* strain has the Δ*adhE* Δ*ldhA* Δ*iclR Δackpta* PYC genotype.

7. The method according to claim 6, **characterized in that** the *Escherichia coli* strain is the SBS550MG-pHL413 strain.

8. A method for obtaining succinic acid, comprising:
- a method for producing succinic acid and/or succinate ions according to any one of claims 1-7;
- where appropriate, acidification of the succinate ions so as to give succinic acid;
- a step of purification of the succinic acid; and
- optionally, a step of crystallizing the succinic acid.

9. A method for obtaining succinic acid according to claim 8, in which the step of purification of the succinic acid is an ethanolic purification.

## Patentansprüche

1. Verfahren zur Herstellung von Bernsteinsäure und/oder Bernsteinsäureionen durch Fermentation eines *Escherichia coli*-Stammes unter anaeroben Bedingungen, umfassend:
(A) einen Schritt der Fermentation einer Kohlenstoffquelle in einem Fermenter mit CO₂-Zufuhr, umgesetzt mittels Lüftungsöffnungen des geschlossenen Fermenters, so dass die CO₂-Zufuhr gemäß dem CO₂-Verbrauch durch den Stamm reguliert ist; nach dem Aufbrauchen der gesamten Kohlenstoffquelle gefolgt von
(B) einem Schritt der Fermentation der verbleibenden Kohlenstoffquelle ohne CO₂-Zufuhr, um das restliche CO₂ zu verbrauchen.

2. Verfahren gemäß Anspruch 1, wobei am Anfang von Schritt (A) die Fermentationsumgebung mit CO₂ gesättigt ist.

3. Verfahren gemäß einem der Ansprüche 1-2, wobei Schritt (A) und/oder Schritt (B) bei einem pH-Wert im Bereich von 6,0-7,0 stattfindet.

4. Verfahren gemäß einem der Ansprüche 1-2, wobei Schritt (A) und/oder Schritt (B) bei einem pH-Wert von 6,4-6,8 stattfindet.

5. Verfahren gemäß einem der Ansprüche 1-4, wobei die Kohlenstoffquelle Glucose ist, und wobei:
- am Anfang von Schritt (A) die Fermentationsumgebung 15-40 g/l Glucose enthält und
- am Anfang von Schritt (B) die Fermentationsumgebung 2-6 g/l Glucose enthält.

6. Verfahren gemäß einem der Ansprüche 1-4, wobei der *Escherichia coli*-Stamm den Genotyp Δ*adhE* Δ*ldhA* Δ*icIR* Δ*ackpta* PYC besitzt.

7. Verfahren gemäß Anspruch 6, **dadurch gekennzeichnet, dass** der *Escherichia coli*-Stamm der Stamm SBS550MG - pHL413 ist.

8. Verfahren zur Gewinnung von Bernsteinsäure, umfassend:
- ein Verfahren zur Herstellung von Bernsteinsäure und/oder Bernsteinsäureionen gemäß einem der Ansprüche 1-7;
- gegebenenfalls eine Ansäuerung der Bernsteinsäureionen zu Bernsteinsäure;
- einen Schritt der Reinigung der Bernsteinsäure; und
- gegebenenfalls einen Schritt der Kristallisation der Bernsteinsäure.

9. Verfahren zur Gewinnung von Bernsteinsäure gemäß Anspruch 8, **dadurch gekennzeichnet, dass** der Schritt der Reinigung der Bernsteinsäure eine ethanolische Reinigung ist.
